# EUROPEAN PATENT APPLICATION

(11) **EP 4 684 789 A1**
(43) Date of publication of application: **28.01.2026**
(21) Application number: 24190939.9
(22) Date of filing: 25.07.2024
(51) Int. Cl.: A61K 33/06, A61K 33/08, A61K 45/06, A61P 1/02

(54) **MICRONIZATION-ACTIVATED CLINOPTILOTITE (PMA-ZEOLITE) FOR THE TREATMENT AND PREVENTION OF ORAL INFLAMMATORY CONDITIONS**

(71) Applicant: Hraschan, Jakob, 9580 Drollobach am Faakersee (AT)
(72) Inventor: Hraschan, Jakob, 9580 Drollobach am Faakersee (AT)
(74) Representative: Kraus & Lederer PartGmbB

(57) **Abstract**

The present invention relates to an oral care composition comprising a micronization-activated clinoptilotite and one or more pharmaceutically acceptable excipients for use in the treatment and/or prevention of an oral inflammatory condition and/or an oral infection. Preferably, the composition is for the use in the treatment and/or prevention of an acute or chronic periodontal disease, preferably periodontitis.

## Description

### Field of the invention

The present invention relates to the field of dentistry and, in particular, to the treatment and prevention of oral inflammatory conditions and/or oral infections, in particular periodontal diseases, gingivitis, mucositis, periodontitis and periimplantitis as well as endodontic treatment. The invention relates particularly to the PMA-Zeolite intervention for treatment of periodontal diseases caused by bacteria, *i.e.* to the reduction or elimination of the symptoms associated with periodontal diseases. The present invention provides an oral care composition comprising a micronization-activated clinoptilotite (PMA-Zeolite), for example, in toothpaste or as a suspension in buffered saline solution or the like for use in regular stomatology prophylaxis and personal treatment of periodontal disease. The treatment aims at reducing or eliminating the symptoms of periodontal diseases which are caused by certain types of bacteria.

### Background of invention

Cardinal symptoms of periodontal diseases are loss of periodontal tissue attachment, loss of alveolar bone and pocket formation (Highfield J., Diagnosis and classification of periodontal disease. Aust. Dent. J. 2009; 54 Suppl 1:S11-26. doi:10.1111/j.1834-7819.2009.01140.x). The severity of the periodontal disease depends on environmental and host risk factors, both modifiable (for example, smoking) and non-modifiable (for example, genetic susceptibility) (Kinane DF, Stathopoulou PG, Papapanou PN. Periodontal diseases. Nat Rev Dis Primers. 2017 Jun 22; 3:17038).

Gingivitis is the mildest form of periodontal disease and can be found in up to 90% of the population. Periodontitis occurs upon progression of gingivitis into a chronic, destructive, irreversible inflammatory disease state when bacteria penetrate deeper into the tissues and surrounding periodontium causing destruction of the periodontium, alveolar bone loss, and eventually loss of the affected tooth (Gasner NS, Schure RS. Periodontal Disease. 2023 Apr 10. In: StatPearls. Treasure Island (FL): StatPearls Publishing; 2024).

Periodontal diseases are classified as gingival lesions, periodontitis, periodontitis as a manifestation of systemic diseases and developmental and acquired conditions and peri-implant diseases and conditions (Babay N, Alshehri F, Al Rowis R. Majors highlights of the new 2017 classification of periodontal and peri-implant diseases and conditions. Saudi Dent J. 2019 Jul; 31 (3): 303-305. doi:10.1016/j.sdentj.2019.04.006)

Prevention of periodontal diseases is based on oral hygiene maintenance through self-care and professional maintenance through dental cleaning and biofilm removal at scheduled follow-ups (Kinane DF, Stathopoulou PG, Papapanou PN. Periodontal diseases. Nat Rev Dis Primers. 2017 Jun 22; 3:17038).

Treatment of this condition relies on recognition of the pathogenic role of bacteria and their accumulation in the periodontal pocket and oral cavity. A ground-breaking experiment in humans suggested a cause-effect relationship between the aggregation of bacterial deposits in the area of the gingival crevice and gingival inflammation (Andrea Mombelli (2000) Microbial colonization of the periodontal pocket and its significance for periodontal therapy Periodontology 2000 76, 2018, 85-96). It proved that as a consequence of the absence of oral hygiene, bacteria multiply to form macroscopically visible deposits on the teeth. Within 9 to 21 days, clinical signs of gingivitis appeared. When the bacterial deposits were removed and tooth cleaning was resumed, gingivitis subsided. The postulate that subgingival microbial aggregates cause periodontitis is an extrapolation of the finding that bacterial deposits at the gingival crevice cause gingivitis. As a result of gingival swelling and attachment loss, pocket depth increases, and an anaerobic subgingival microbiota concomitantly evolves.

It is assumed that gingivitis converts to periodontal diseases when complex bacterial interactions overload the local host resistance mechanisms. Even though bacteria cause suppuration and loss of bone, antimicrobial agents alone cannot solve these problems (Andrea Mombelli (2000) Microbial colonization of the periodontal pocket and its significance for periodontal therapy Periodontology 2000 76, 2018, 85-96).

Studies have also shown that suspected periodontal pathogens prevail in oral sites other than the periodontal pocket if the micro-environmental conditions are favourable. Accordingly, in subjects free of periodontal disease, *Prevotella intermedia, Aggregatibacter actinomycetemcomitans* and other suspected periodontal pathogens were recovered from deep periodontal sites of third molars under normal eruption (Mombelli A, Buser D, Lang NP, Berthold H. Suspected periodontopathogens in erupting third molar sites of periodontally healthy individuals. J Clin Periodontol 1990: 17: 48-54).

By use of a specially designed nozzle introduced into a periodontal pocket, it is possible to remove subgingival nonmineralized bacterial deposits with a jet of compressed air containing a light abrasive and beneficial shifts in the composition of the subgingival microbiota may occur over certain period in moderate-to-deep periodontal pockets (McNabb H, Mombelli A, Lang NP. Supragingival cleaning three times a week: the microbiological effects in moderately deep pockets. J Clin Periodontol 1992: 19: 348-356).

In classic bacterial infections the diversity of the microbiota decreases as the disease develops; in most cases of periodontitis, however, the diversity of the flora increases (Dekic S, Hrenovic J, Hunjak B, Kazazic S, Tibljas D, Ivankovic T (2017) Virulence Factors of Acinetobacter baumannii Environmental Isolates and Their Inhibition by Natural Zeolite. Int.J.Curr.Microbiol.App.Sci 6 (3): 1697-1709).

Current periodontal therapy is based on interventions applied in a stepwise approach with the goal to control the infection and counteract the inflammation process (Ridgeway EE. Periodontal disease: diagnosis and management. J Am Acad Nurse Pract. 2000 Mar; 12 (3):79-84; Sanz M, Herrera D, Kebschull M, Chapple I, Jepsen S, Beglundh T, et al. Treatment of stage I-III periodontitis-The EFP S3 level clinical practice guideline. J Clin Periodontol. 2020 Jul; 47 (S22 Suppl 22):4-60. https://doi.org/10.1111/jcpe.13290)

The first step of periodontal therapy is based on removal of the supragingival biofilm, on control of proven risk factors in the etiopathogenesis of periodontal diseases. This step is used in patients with diagnosed gingivitis. The second step is done in chronic or persistent cases and comprises removal of subgingival biofilm and calculus by subgingival instrumentation with or without adjunctive local/systemic antimicrobial or anti-inflammatory medications. If these steps fail to provide clinical benefits, the third step is applied which comprise repeated subgingival instrumentation or other periodontal surgical interventions (Nogueira A, Furlaneto F, Gaio E-J, Gomes SC, Palioto DB, Castilho RM, Sanz M, Messora MR. Newtendencies in non-surgical periodontal therapy. Braz. oral. res. 35 (Supp 2). 2021: e095).

Chronic periodontal disease treatment may be done by use of antimicrobial therapy by systemic and/or locally administered antibiotics depending on the severity of the disease (Barca E, Cifcibasi E, Cintan S. Adjunctive use of antibiotics in periodontal therapy. J Istanb Univ Fac Dent. 2015; 49 (3): 55-62; Kaner D, Christan C, Dietrich T, Bernimoulin JP, Kleber BM, Friedmann A. Timing affects the clinical outcome of adjunctive systemic antibiotic therapy for generalized aggressive periodontitis. J Periodontol 2007: 78: 1201-1208; Killoy WJ. The clinical significance of local chemotherapies. J Clin Periodontol 2002: 29: 22-29).

Antibiotics can be employed as adjunctive therapy in chronic periodontic disease (Smiley CJ, Tracy SL, Abt E, Michalowicz BS, John MT, Gunsolley J, Cobb CM, Rossmann J, Harrel SK, Forrest JL, Hujoel PP, Noraian KW, Greenwell H, Frantsve-Hawley J, Estrich C, Hanson N. Systematic review and meta-analysis on the nonsurgical treatment of chronic periodontitis by means of scaling and root planing with or without adjuncts. J Am Dent Assoc 2015: 146: 508-524; Mombelli A, Cionca N, Almaghlouth A. Does adjunctive antimicrobial therapy reduce the perceived need for periodontal surgery? Periodontol 2000. 2011 Feb; 55 (1): 205-16. doi:10.1111/j.1600-0757.2010.00356.x).

Published data show that local antibiotic therapy value has decreased in recent years as a majority of adequately tested formulations have been withdrawn from the market for different reason, including economic reasons (Mombelli A, Cionca N, Almaghlouth A. Does adjunctive antimicrobial therapy reduce the perceived need for periodontal surgery? Periodontol 2000. 2011 Feb; 55 (1): 205-16. doi:10.1111/j.1600-0757.2010.00356.x).

Systemic antimicrobials are used adjunctively to mechanical debridement, preferably as part of nonsurgical periodontal therapy (Sanz M, Teughels W; Group A of European Workshop on Periodontology. Innovations in non-surgical periodontal therapy: Consensus Report of the Sixth European Workshop on Periodontology. J Clin Periodontol. 2008 Sep; 35 (8 Suppl): 3-7. doi: 10. 1111/j.1600-051X.2008.01256.x)

Most applied systemic antibiotic treatments for chronic periodontic disease include Tetracycline, Doxycycline, Minocycline, Metronidazole, Amoxicillin + Clavulinic acid (Augmentin), Metronidazole + Amoxicillin, Ciprofloxacin plus Metronidazole, Azithromycin (Tonetti MS, D'Aiuto F, Nibali L, Donald A, Storry C, Parkar M, Suvan J, Hingorani AD, Vallance P, Deanfield J. Treatment of periodontitis and endothelial function. N Engl J Med 2007: 356: 911-920; Kapoor A, Malhotra R, Grover V, Grover D. Systemic antibiotic therapy in periodontics. Dent Res J (Isfahan). 2012 Sep; 9 (5): 505-15. doi:10.4103/1735-3327.104866).

Periodontitis has recently been growingly acknowledged as an inflammatory disease of oxidative stress and therapeutic options have been expanded on the usage of antioxidant compounds such as for example resveratrol and/or modulators of host innate immunity such as for example probiotics as well (Sczepanik FSC, Grossi ML, Casati M, Goldberg M, Glogauer M, Fine N, Tenenbaum HC. Periodontitis is an inflammatory disease of oxidative stress: We should treat it that way. Periodontol 2000. 2020 Oct; 84 (1): 45-68. doi:10.1111/prd.12342; Allaker RP, Douglas CW. Novel antimicrobial therapies for dental plaque-related diseases. Int J Antimicrob Agents. 2009; 33 (1): 8-13; Iniesta M, Herrera D, Montero E, Zurbriggen M, Matos AR, Marín MJ, Sánchez-Beltrán MC, Llama-Palacio A, Sanz M. Probiotic effects of orally administered Lactobacillus reuteri-containing tablets on the subgingival and salivary microbiota in patients with gingivitis. A randomized clinical trial. J Clin Periodontol 2012: 39: 736-744).

Newer strategies to target formation of biofilms by periodontal bacteria with peptidomimetic agents are also studied (Tan J, Patil PC, Luzzio FA, Demuth DR: In Vitro and In Vivo Activity of Peptidomimetic Compounds That Target the Periodontal Pathogen Porphyromonas gingivalis. Antimicrob Agents Chemother 2018, 62 (7)).

Different allumosilicates (zeolites) proved efficient in inhibition of biofilm formation in nanocomposite form (ZnO/ZeoNC) against standard and clinical strains of *Staphylococcus aureus* (Partoazar, A., Bideskan, F.R., Partoazar, M. Talaei, N., Dallal M.M.S. Inhibition of Biofilm Formation of Staphylococcus aureus Strains Through ZnO/Zeolite Nanocomposite and Its Cytotoxicity Evaluation. BioNanoSci. 10, 714-720 (2020). https://doi.org/10.1007/s12668-020-00761-x), in zeolite silver-impregnated clay granular and silver impregnated porous pot filtration system formulation (Budeli P, Moropeng RC, Mpenyana-Monyatsi L, Momba MNB. Inhibition of biofilm formation on the surface of water storage containers using biosand zeolite silver-impregnated clay granular and silver impregnated porous pot filtration systems. PLoS One. 2018 Apr 5; 13 (4): e0194715) or in the silver-lon-Exchanged Zeolite A against *Vibrio spp* marine pathogens biofilm formation (Amin Z, Waly NA, Arshad SE. Biofilm Inhibition and Antimicrobial Properties of Silver-lon-Exchanged Zeolite A against Vibrio spp Marine Pathogens. Appl. Sci. 2021, 11 (12), 5496; https://doi.org/10.3390/app11125496).

The allumosilicate Zeolite 4A can also interfere with bacterial communication and disrupt biofilm formation in *Pseudomonas aeruginosa-related* infections (Ulusoy S, B Akalin R, Çevikba 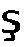 H, Berisha A, Oral A, Bo 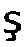 geimez-Tinaz G. Zeolite 4A as a jammer of bacterial communication in Chromobacterium violaceum and Pseudomonas aeruginosa. Future Microbiol. 2022 Jul; 17:861-871).

The natural allumosilicate zeolite clinoptilolite, also strongly inhibits biofilm formation and twitching motility of bacteria due to immobilization of bacterial cells onto zeolite particles (Dekic S, Hrenovic J, Hunjak B, Kazazic S, Tibljas D, Ivankovic T (2017) Virulence Factors of Acinetobacter baumannii Environmental Isolates and Their Inhibition by Natural Zeolite. Int. J. Curr. Microbiol. App. Sci. 6 (3): 1697-1709).

### Disclosure of the invention

### Problem to be solved by the invention

An object of the present invention is to provide new compositions/means for treating/preventing oral inflammatory conditions and/or oral infections, in particular means for treating of inflammation-driven periodontal diseases in a patient, wherein the periodontal state (diseases) is caused by change in bacteria population in the oral cavity.

An object of the present invention is thus to provide oral care compositions for treating and/or preventing an oral inflammatory condition and/or an oral infection. The oral care composition is preferably suitable for the treatment of inflammation-driven periodontal diseases in a patient wherein the periodontal state (diseases) is caused by a change in the bacteria population in the oral cavity.

In particular, the oral care compositions are to reduce or eliminate symptoms associated with an oral inflammatory condition and/or an oral infection, preferably, to reduce or eliminate the symptoms associated with periodontal diseases.

The compositions are to be used alone or in combination with standard treatments of an oral inflammatory condition and/or an oral infection, in particular, in combination with standard periodontal treatments.

Preferably, the compositions are to be more effective and/or have fewer side effects than known compositions for treating oral inflammatory conditions and/or an oral infection, in particular, for treating periodontal diseases. The compositions, preferably, have no side effects and are easy to applicate and are suitable to be used by a variety of the population, i.e. also for children and the elderly. The composition should also be suitable for self-treatment.

### Summary of the invention

The inventors' study towards achieving the above objects unexpectedly found that a micronization-activated clinoptilotite (in the following also proprietary PMA-zeolite, alumosilicate) exhibits remarkable therapeutic effect and reduces or eliminates the symptoms associated with the simultaneous syndromes known as periodontal diseases caused by exposition to certain bacteria and/or bacterial disbalance in the oral cavity, including dental pockets. The micronization-activated clinoptilotite is also suitable for the treatment of a variety of oral inflammatory conditions and/or oral infections. Hence the present invention relates to the non-cosmetic use of the micronization-activated clinoptilotite and provides the following:

An oral care composition comprising a micronization-activated clinoptilotite and one or more pharmaceutically acceptable excipients for use in the treatment and/or prevention of an oral inflammatory condition and/or an oral infection.

The micronization-activated clinoptilotite is a zeolite particle composition obtained by a process according to EP 3 329 926. This zeolite is also referred to as PMA zeolite. PMA is the abbreviation for "Panaceo Micro Activation" and has become an accepted designation for this type of zeolite in the art. The starting material is the naturally occurring zeolite clinoptilotite which is treated according to the process of EP 3 329 926 (see below).

Preferably, the oral care composition for the above use is used for the treatment of an acute or chronic periodontal disease, a peri-implant disease, an oral wound and/or an aphthous lesion.

Preferably, the periodontal disease is gingivitis, oral mucositis and/or periodontitis.

Preferably, the oral care composition for the above use is for the treatment of a bacterial, viral or fungal infection.

Preferably, the oral care composition is a composition selected from a mouthwash, a toothpaste, a powder, a dental gel, a chewing gum, a dissolvable, partially dissolvable or non-dissolvable film or strip or a dental floss.

Preferably, the oral care composition comprises further ingredients such as one or more of a fluoride ion source, breath-freshening agents, antiplaque agents, analgesics, nutrients, abrasives, surfactants, thickening agents, buffers, preservatives and antibiotic and anti-caries agents.

Preferably the oral care composition is a toothpaste having a content of micronization-activated clinoptilotite of 2% to 10% wt/wt. In more preferred embodiments, the composition is a toothpaste having a content of micronization-activated clinoptilotite of 2% wt./wt. to 5% wt./wt or a toothpaste having a content of micronization-activated clinoptilotite of 5-10% wt./wt.

Preferably, the oral care composition is provided in a package containing powdered micronization-activated clinoptilotite for the preparation of a suspension of oral cavity rinsing including periodontal pockets. Preferably the treatment is within a regular daily oral hygiene maintenance regimen.

### Detailed Description of Invention

The oral care composition provided by the present invention can be used for the treatment and/or prevention of oral inflammatory conditions and/or oral infections. Oral inflammations and infections can basically affect different parts of the mouth including the gums, tongue, lips and the lining of the teeth. Common examples of such inflammatory conditions are gingivitis, stomatitis, mucositis and/or periodontitis. Preferably, the oral care composition as disclosed herein is used for the treatment of periodontal diseases. In the context of invention, periodontal diseases are caused by inflammation induced by chronic bacterial infection and result in the destruction and loss of periodontal tissue. The invention achieves in particular, a reduction or elimination of periodontal process. The process may be acute (beginning suddenly) or chronic (developing gradually, often over months or years). Chronic infection and inflammation of periodontal tissue affects the establishment or complication of systemic diseases such as diabetes mellitus, chronic kidney disease, blood vessel disorder, non-alcoholic steatohepatitis, and rheumatoid arthritis.

The active ingredient of the oral care compositions for use according to the invention is a micronization-activated clinoptilotite. By a micronization process, a zeolite/alumosilicate is reduced in particle size to less than 10 microns. The size reduction by micronization can, for example, be achieved by acceleration of particles so that grinding occurs, by particle-to-particle impact or impact against a solid surface. In fluid energy mills (also known as jet mills), micronization is achieved by high-impact velocity as a result of particle acceleration in a fast gas stream. In a jet mill, particle velocities are in the range of 300 to 500 m/sec compared to 50 to 150 m/sec in a mechanical impact mill. The micronization process is associated with advantages such as increased bioavailability, increased surface area and bioequivalence. According to the present invention, the zeolite clinoptilotite is used and the micronization-activated clinoptilotite is prepared by a method according to EP 3 329 926. This method comprises the steps of
(a) providing natural zeolite particles containing at least 85 wt.% of clinoptilolite as determined by X-ray diffraction and having a volume-based particle size as measured by laser diffraction ranging between 0.001 and 0.5 mm;
(b) introducing the particles provided in step (a) in a first micronization device via a particle inlet tube (6) and tribo-mechanically micronizing the particles;
(c) collecting the particles micronized in step (b) at a particle outlet port of the first micronization device; (d) introducing the particles collected in step (c) in the first or a second micronization device via a particle inlet tube (6) and tribo-mechanically micronizing the particles; and (e) collecting the particles micronized in step (d) at a particle outlet port of the second micronization device;
(f) optionally repeating steps (d) and (e);
wherein the particles are treated with a gas containing at least 95 mol-% of oxygen throughout at least one of steps (b) or (d).

The zeolite particle composition obtained by the above method is characterized by particles having a specific surface area of 30.5 to 30.8 m²/g, wherein the specific surface area is measured by multipoint BET surface area measurement and an average of zeta potential values of less than -12.00 mV, wherein the zeta potential values are measured after addition of Ni²⁺ ions at a concentration of 0.001 M to 50 ml of an aqueous composition containing 50 mg of zeolite particles at different pH values in the pH range of 3.2 to 6.0 where the different pH values are achieved by adding 0.1 M HCl in aliquots of 20 ml to the aqueous composition and wherein the zeta potential values are calculated based upon experimentally determined electrophoretic mobility. Preferably, the particles are further characterized by a silicon to aluminum ratio at the surface of 4.0 to 5.5, wherein the ratio is measured by Energy Dispersive X-ray Diffraction at an accelerating voltage of 15 kV. The zeolite to be used according to the present invention is in the following also termed proprietary PMA-Zeolite or alumosilicate.

The proprietary PMA-Zeolite used according to the present invention is used to treat any oral inflammatory conditions and/or oral infections, preferably to treat conditions caused by bacteria and/or their disbalance in the oral cavity that cause periodontal diseases. The micronization-activated clinoptilotite is used within a usual formulation for oral care products or a pharmaceutical formulation to be used in the area of the mouth.

A pharmaceutical composition/ oral care formulation/oral care composition for the use according to the invention can be prepared by common procedures using a conventional pharmaceutical/oral care carrier, excipient, or the like, which are known to a person skilled in the art. The composition may be solid or liquid suspension, gel or the like and toothpaste.

The composition for the use of the invention may be administered in any way in which oral care compositions, i.e. compositions having effect in the oral cavity, are typically administered to a patient. This may be oral rising, brushing with a toothpaste, swishing or gargling, direct application to affected areas, flossing between teeth, chewing of a chewing gum, cleaning with interdental brushes, water-jet cleaning, spraying, etc. Accordingly, the oral care composition may be in the form of a suspension, a gel, a powder, a gum formulation or a toothpaste. The composition may also be provided in the form of a PMA-Zeolith paste to be mixed with water. These compositions contain pharmaceutically acceptable ingredients commonly used in said administration forms and are known to a person skilled in the art.

As a supportive solid composition for oral administration according to the invention a powder or capsule may be used. In such a solid composition, one or more active ingredients are mixed with, at least one inert diluent such as lactose, mannitol, glucose, hydroxypropyl cellulose, microcrystalline cellulose, starch, polyvinyl pyrrolidone, and/or magnesium metasilicate aluminate. These forms are to be dissolved in water or any other suitable rinsing solution.

A liquid or solid composition for direct topical administration may include a pharmaceutically acceptable suspension or powder and contains a commonly used inert diluent (in a case of liquid) such as purified water, saline buffered solution or the like.

According to the invention, the proprietary PMA-Zeolite may be administered to a patient with periodontal disease. A suitable orally applied daily dose ranges from about 0.01 to 1000 mg/kg of body weight, preferably from 0.1 to 500 mg/kg of body weight, more preferably from 10 to 100 mg/kg of body weight. The daily dose is administered/applied orally or topically once per day or two to four times per day by dividing it into two or four portions. The dose is appropriately determined depending on the individual patient.

According to the invention, the proprietary PMA-Zeolite may be administered to a patient with periodontal disease locally during stomatology procedures and in the form of toothpaste for daily oral hygiene maintenance or in the form of a rinsing solution. A toothpaste preferably has a content of micronization-activated clinoptilotite of 2% to 10% wt/wt, more preferably 2% wt./wt. to 5% wt./wt or 5-10% wt./wt micronization-activated clinoptilotite..

The rinsing dosage is preferably 1 g to 5 g, more preferably 2 g of PMA-Zeolite per 2 dl in total of 500mL of rinsing suspension, preferably demineralized water. The rising dosage for the oral cavity is coupled to a minimum of two toothpaste usage/daily for one to three months but this can be extended to a routine toothpaste usage. A liquid composition for oral administration includes a pharmaceutically acceptable suspension, and the like, and contains a commonly used inert diluent such as purified water. The liquid composition may further contain an auxiliary agent such as a wetting agent, or a suspending agent, a sweetener, a flavour, a perfume, or a preservative other than the inert diluent. The dosage of proprietary PMA-Zeolite in the toothpaste is preferably 5%. Apart from PMA-Zeolite the toothpaste composition may contain Glycerin, Aqua, Hydrated Silica, Aloe Barbadensis Leaf Juice, Mentha Piperita Water, Sodium Coco-Sulfate, Xanthan Gum, Zinc Oxide, Mentha Spicata Herb Oil, Mentha Avensis Leaf Oil, Chondrus Crispus Powder, Stevia Rebaudiana Leaf/Stem Extract, Citric Acid, Potassium Sorbate, Sodium Benzoate, Limonene. The composition may further comprise one or more of a fluoride ion source, breath freshening agents and antiplaque agents, analgesics, nutrients, abrasives, surfactants, thickening agents, buffers, preservatives and antibiotic as well as anticaries agents. The composition may also contain further pharmaceutically active ingredients. The composition may also be in the form of a paste to be mixed with water. In this case a paste dosing of 4 g PMA- Zeolite is mixed with 1 to 1,5, ml water.

The patient may be any mammal, preferably a human being. The composition may, however, also be administered to pet animals, such as dogs or cats. The patient is, in particular, a patient already suffering from periodontal disease. The composition may, however, also be administered as a prophylactic composition.

The proprietary PMA-Zeolite may be administered, in particular, to a patient at any stage of periodontal disease. It may be administered together with an antibiotic. It may be administered within the daily oral care routine or according to a prescribed therapeutic regimen. The composition may be administered over a predetermined time period or over a longer time period such as several weeks, several months, a year or longer. The composition may also be administered in a cyclic dosing regimen with treatment-free intervals. This may be particularly relevant for a prophylactic treatment. The composition may be administered as a long-term prophylactic treatment or only when therapeutically needed and prescribed by a medical doctor. The composition is also suitable for self-medication.

The administration of the proprietary PMA-Zeolite according to the invention, provides excellent effects on reducing symptoms or eliminating a periodontal disease.

### Brief description of the drawings

Figure 1: Summary of the study course and PMA-Zeolite treatment plan.
Figure 2: Enriched phyla in all patients with diagnosed *periodontitis chronico* in the study and assessed by whole-genome sequencing were *Proteobacteria, Firmicutes, Bacterioidetes, Actinobacteria, Synergistetes, Spirochaetes, Fusobacteria and Chlorofelxi* (ANOVA, Tukey-Kramer, eta squared, p<0.05).
Figures 3a to 3h: General comparison of the microbiome status in all tested patient samples before and after therapy

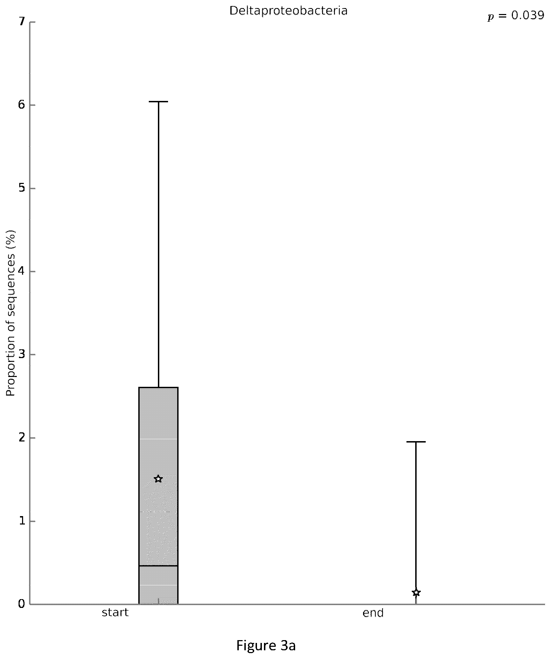

Figure 3a: For the class comparison - Deltaproteobacteria; Based on phylogenetic analysis of 16S rRNA gene the Proteobacteria phylum is divided into 6 classes (previously regarded as subclasses of the phylum): Alphaproteobacteria, Betaproteobacteria, Gammaproteobacteria, Deltaproteobacteria, Epsilonproteobacteria, and Zetaproteobacteria. https://www.hindawi.com/journals/bmri/2017/9351507/.

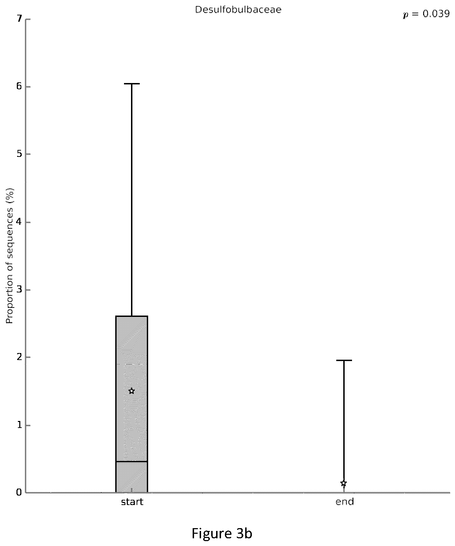

Figure 3b: For the family comparison - Desulfobulbaceae; Relative abundance Desulfobulbaceae is higher in parodontic patients, https://www.frontiersin.org/articles/10.3389/froh.2021.722495/full.

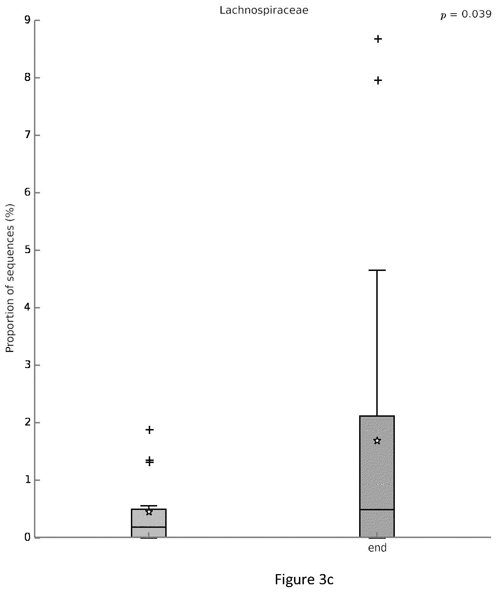

Figure 3c: For the family comparison - Lachnospiraceae; For Lachnospiraceae isolates from 20 human donors aan enormous genetic and functional inter- and intraspecies diversity in the Lachnospiraceae family was observed showing that probably impacts beneficial functions such as butyrate production and lantibiotic expression. Lantibiotics produced by a specific *Blautia producta* strain can reduce colonization by vancomycin-resistant Enterococcus (https://journals.lww.com/cogastroenterology/Fulltext/2021/11000/Gut_microbiota_and_sepsis_fr om_pathogenesis_to.7.aspx ).
   The Lachnospiraceae family is formed by 24 named genera, including Ruminococcus, Blautia, Dorea, and Lachnoanaerobaculum as well as a number of incertae sedis strains. https://www.frontiersin.org/articles/10.3389/fped.2016.00057/full
   Lachnospira is a signature for predicting the efficacy of antihistamine in patients with chronic spontaneous urticaria, https://pubmed.ncbi.nlm.nih.gov/34558729/
   Recent studies underline the importance of understanding the biological role of the Lachnospiraceae family and, in particular of R. gnavus, in the complexity of the human microbiota in babies and adults. https://www.frontiersin.org/articles/10.3389/fped.2016.00057/full.

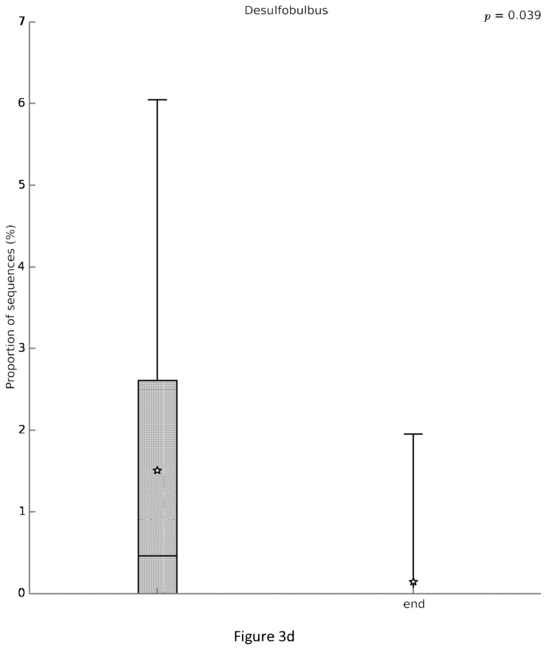

Figure 3d: For the genus comparison - Desulfobulbus

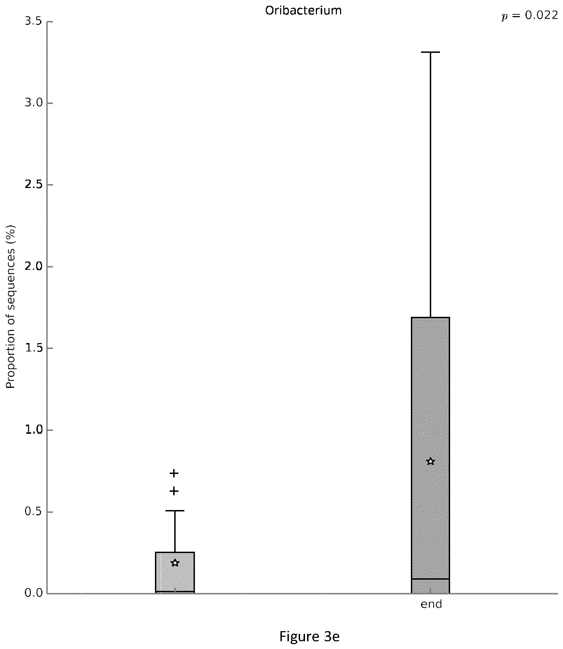

Figure 3e: For the genus comparison - Oribacterium; Oribacterium. Oribacterium is a strictly anaerobic and non- spore -forming bacterial genus from the family of Lachnospiraceae Oribacteria was not changed in one study of first phase of orthodontic treatment of patients with parodontosis https://ss.bjmu.edu.cn/Sites/Uploaded/File/2022/02/186378076933717470944708676.pdf.

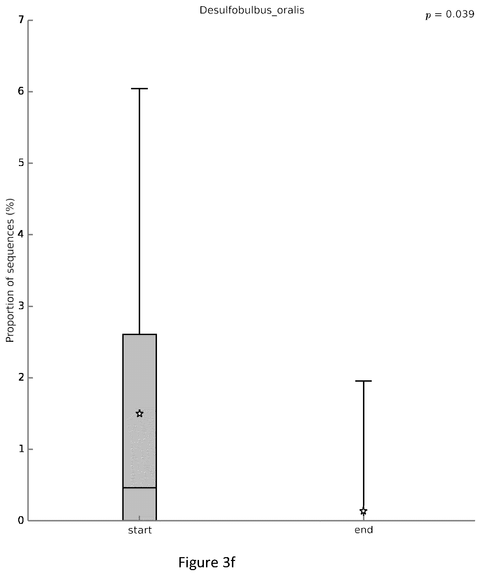

Figure 3f: For the species comparison - Desulfobulbus oralis; Karissa L. Cross, Payal Chirania, Weili Xiong, Clifford J. Beall, James G. Elkins, Richard J. Giannone, Ann L. Griffen, Adam M. Guss, Robert L. Hettich, Snehal S. Joshi, Elaine M. Mokrzan, Roman K. Martin, Igor B. Zhulin, Eugene J. Leys and Mircea Podar (2018) Insights into the Evolution of Host Association through the Isolation and Characterization of a Novel Human Periodontal Pathobiont, Desulfobulbus oralis, mBio 9(2):10.1128/mbio.02061-17; Cai Z, Lin S, Hu S, Zhao L. Structure and Function of Oral Microbial Community in Periodontitis Based on Integrated Data. Front Cell Infect Microbiol. 2021 Jun 17;11:663756. doi: 10.3389/fcimb.2021.663756.

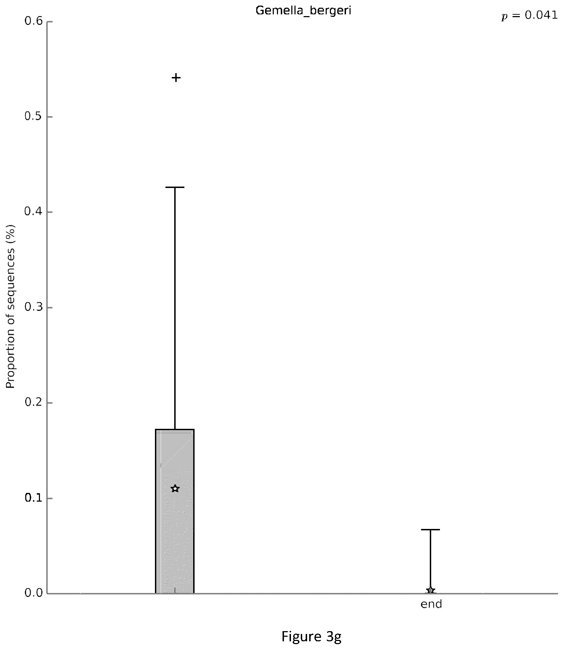

Figure 3g: For the species comparison - Gemella bergeri; Lim KR, Son JS, Moon SY. The First Case of Infectious Spondylitis Caused by Gemella bergeri. Medicina (Kaunas). 2023 Jan 11;59(1):145.

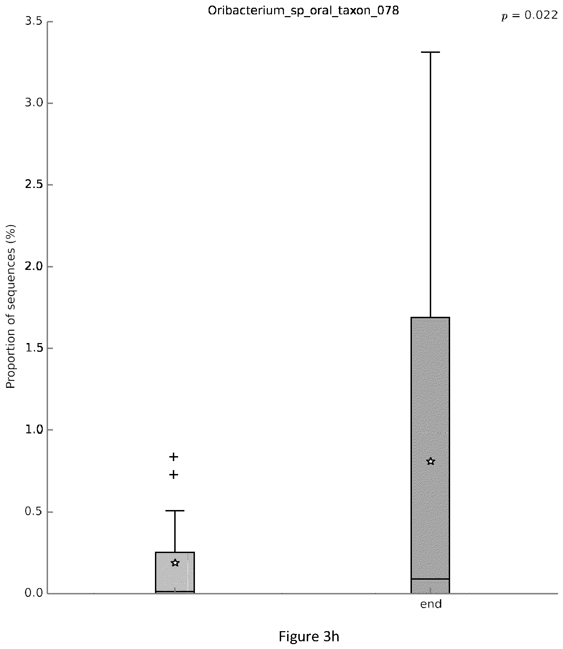

Figure 3h: For the species comparison - Oribacterium sp.; Talita Gomes Baeta Louren 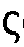 o, Sarah J. Spencer, Eric John Alm, Ana Paula Vieira Colombo(2018) Defining the gut microbiota in individuals with periodontal diseases: an exploratory study. Journal of Oral Microbiology Volume 10(1), https://doi.org/10.1080/20002297.2018.1487741.

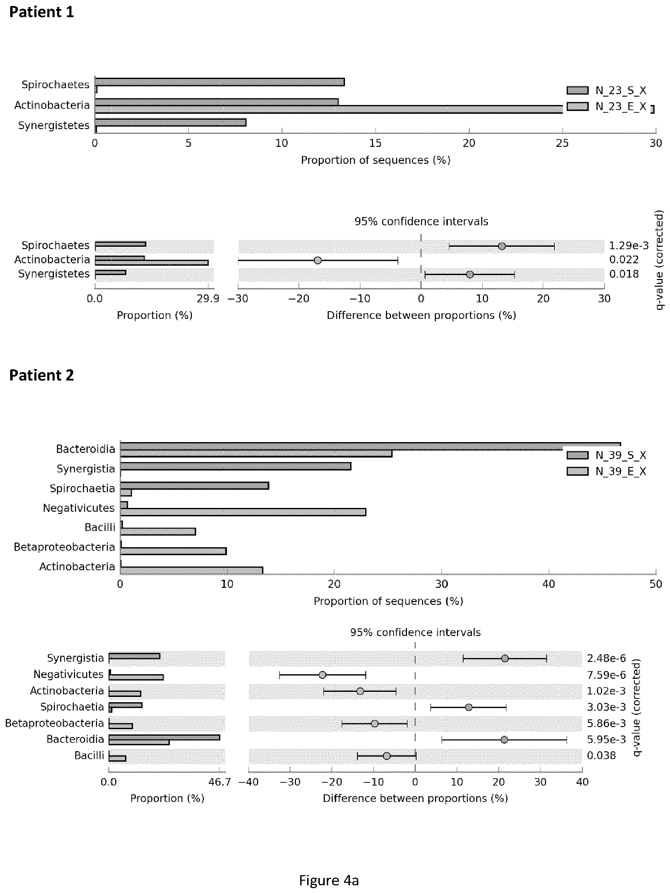

Figures 4a to 4d: Individual statistically relevant class differences in 7 patients. The two-sided Fisher's exact test with Benjamini-Hochberg FDR correction was used, p<0.05) showed a highly individualized response in class comparison due to large oral microbiome variations already documented in the literature. These individual data is presented for each patient.

The present invention is illustrated by the following example.

### Examples

Example 1: Application of proprietary PMA-Zeolite on the microbial colonization in the treatment of periodontitis.

In a clinical study, efficacy of the proprietary PMA-Zeolite in the form of suspension for oral rising during stomatology examinations and standard treatment procedures and in the form of a toothpaste for maintenance of daily oral hygiene, was assessed. The monitored outcomes included effects on microbial colonization in periodontitis during 10 weeks interventional period on 37 patients with the diagnosis of periodontitis chronica. Patients were sampled at the beginning of the study, at the end of the intervention. Effect of the treatment was also assessed on pocket depth (PD) and bleeding on probing (BoP).

### 1.General Design and Result of the Study

This study was designed as a prospective, randomized, nonblinded, controlled, single centre clinical trial. For the purpose of this research, periodontal evaluation (assessment of oral mucosa, probing depth, bleeding on probing status and plaque index) was performed prior to the enrolment into the study and sampling, during interventional periods and at the end of the treatment.

In the clinical study, the enrolled patients were given plaque-free professional prophylaxis at the beginning. The PMA-Zeolite treatment was performed by use of PMA-Zeolite solution for rinsing of periodontal pockets. Patients were then instructed how to brush their teeth with PMA-Zeolite toothpaste for 2 minutes at home during regular daily oral hygiene (morning and evening) during the whole study period and not to eat and drink for one (1) hour after teeth brushing. The follow-up was done after 12 week-period (2 weeks upon PMA-Zeolite treatment completion) when the patients assessed their subjective feeling as well.

The proprietary PMA-Zeolite treatment showed clear improvement in clinical periodontal indices after local application of PMA-Zeolite toothpaste and rinsing of pathological periodontal pockets with PMA-Zeolite demineralized water solution in all patients after 10 weeks of PMA-Zeolite treatment. The clinical results at the end of PMA-Zeolite therapy showed a marked statistical difference towards improved values for the BoP and PD. The improved clinical state and subjective feeling were also confirmed at the follow-up.

The identified microbiome changes also pointed to the effectiveness of the PMA-Zeolite-based treatment on the entire periodontal microbiome status in the treatment of periodontal disease. The statistically relevant microbiome analysis comparison of ALL samples before and after PMA-Zeolite treatment confirmed specifically decreased abundance of the class Deltaproteobacteria, the families *Desulfobulbaceae* and *Lachnospiraceae,* the genus *Desulfobulbus* and *Oribacterium,* the species *Desulfobulbus oralis* and *Gemella bergeri* that may play a role in periodontal disease and oral cavity inflammation (Rizzatti G, Lopetuso LR, Gibiino G, Binda C, Gasbarrini A. Proteobacteria: A Common Factor in Human Diseases. Biomed Res Int. 2017; 2017:9351507; Esparbès P, Legrand A, Bandiaky ON, Chéraud-Carpentier M, Martin H, Montassier E, Soueidan A. Subgingival Microbiota and Cytokines Profile Changes in Patients with Periodontitis: A Pilot Study Comparing Healthy and Diseased Sites in the Same Oral Cavities. Microorganisms. 2021 Nov 16; 9 (11): 2364; Toyoshima H, Fujii K, Tanigawa M, Nakamura A, Tanabe M, Tanaka H, Nakanishi Y, Sakabe S. The First Case Report of Mediastinal Abscess Caused by Gemella bergeri. Intern Med. 2021 May 15; 60 (10): 1631-1635; Lim KR, Son JS, Moon SY. The First Case of Infectious Spondylitis Caused by Gemella bergeri. Medicina (Kaunas). 2023 Jan 11; 59 (1): 145; Chowdhry A, Kapoor P, Bhargava D, Bagga DK. Exploring the oral microbiome: an updated multidisciplinary oral healthcare perspective. Discoveries (Craiova). 2023 Jun 30; 11 (2): e165; Wolff D, Frese C, Schoilew K, Dalpke A, Wolff B, et al. (2019) Amplicon-based microbiome study highlights the loss of diversity and the establishment of a set of species in patients with dentin caries. PLOS ONE 14 (7): e0219714; Esparbès P, Legrand A, Bandiaky ON, Chéraud-Carpentier M, Martin H, Montassier E, Soueidan A. Subgingival Microbiota and Cytokines Profile Changes in Patients with Periodontitis: A Pilot Study Comparing Healthy and Diseased Sites in the Same Oral Cavities. Microorganisms. 2021 Nov 16; 9 (11):2364; Toyoshima H, Fujii K, Tanigawa M, Nakamura A, Tanabe M, Tanaka H, Nakanishi Y, Sakabe S. The First Case Report of Mediastinal Abscess Caused by Gemella bergeri. Intern. Med. 2021 May 15; 60 (10): 1631-1635; Lim KR, Son JS, Moon SY. The First Case of Infectious Spondylitis Caused by Gemella bergeri. Medicina (Kaunas). 2023 Jan 11; 59 (1): 145; Chowdhry A, Kapoor P, Bhargava D, Bagga DK. Exploring the oral microbiome: an updated multidisciplinary oral healthcare perspective. Discoveries (Craiova). 2023 Jun 30; 11 (2): e165; Wolff D, Frese C, Schoilew K, Dalpke A, Wolff B, et al. (2019) Amplicon-based microbiome study highlights the loss of diversity and the establishment of a set of species in patients with dentin caries. PLOS ONE 14 (7): e0219714).

### 2. Objectives (primary and secondary endpoints)

Primary endpoint:
- To assess the efficacy of PMA-Zeolite on microbial colonization in periodontitis during 10 weeks interventional period

Secondary endpoints:
- To assess the effect of PMA-Zeolite on pocket depth (PD)
- To assess the effect of PMA-Zeolite on bleeding on probing (BoP)
- To assess the effect of PMA-Zeolite on approximal plaque index (API)

### 3. Specific Study Design

This study is designed as a prospective, randomized, nonblinded, controlled, single centre clinical trial.

Study comprised 67 patients randomly assigned to the test group (to be treated with PMAZeolite) and 22 patients assigned to the control group (to receive only standard prophylaxis), all consented outpatients with the diagnosis of *periodontitis chronica.* After initial evaluation, and immediate drop-out of 12 patients due to the earthquake in Zagreb (as described in the results section), 36 patients were assigned to the PMA-Zeolite treatment group and 6 patients were controls that did not receive PMA-Zeolite treatment (totally assigned 42). All study subjects were recruited from the Department of oral medicine and Department of periodontology School of Dental medicine University of Zagreb. Diagnosis of *periodontitis chronica* in all recruited study subjects was established by experienced clinicians, specialists in oral medicine and periodontology according to accepted clinical criteria.

For the purpose of this research, periodontal evaluation (assessment of oral mucosa, probing depth, bleeding on probing status and plaque index) was performed prior to the enrolment into the study and sampling, during interventional periods and at the end of the treatment.

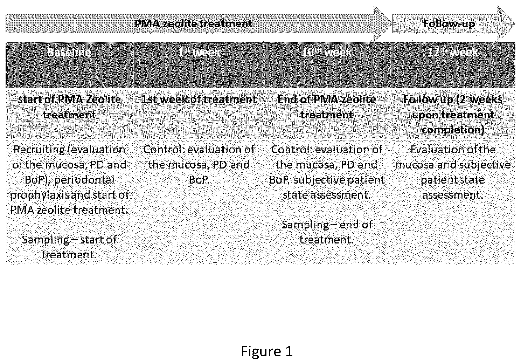

Treatment group patients (n=36) were treated with study medications PMA-Zeolite for up to 10 weeks and were evaluated at baseline, at the end of 1st week, after the 10th week and after 12 weeks (follow-up) (see Figure 1).

Before the experiment, the subjects were given plaque-free professional prophylaxis. They, accordingly, received a thorough teeth cleaning comprising the removal of tarter and plaque build-up. The PMA-Zeolite treatment was performed by use of PMA-Zeolite solution that consisted of 500 ml of freshly prepared demineralized water to which PMA-Zeolite powder was added (ratio zeolite: demineralized water 2g : 2dcl). The periodontal pockets of the test group were rinsed with this solution at the baseline, after 1 week and at the end of study in the dental office. Patients were then instructed how to brush their teeth with PMA-Zeolite toothpaste from Panaceo for 2 minutes at home during regular daily oral hygiene (morning and evening) during the whole study period and not to eat and drink for one (1) hour after teeth brushing.

Six patients from the control group did not undergo PMA-Zeolite treatment but the usual periodontal treatment. In all patients, clinical measurements were performed, and oral saliva samples pooled with periodontal pocket samples were taken for microbiome analysis. For total oral microbiome analysis, 0,5 ml saliva sample and paper point smear from the deepest periodontal pockets were taken in all patients at the baseline and at the end of the treatment.

### 3.1. Patient Population

### Inclusion criteria:

In order to be eligible to participate in the study patients must meet the following criteria:
1. Diagnosis: *periodontitis chronica;* male and female aged 20-70
2. Patients who voluntarily agreed to participate in the study and provide signed and dated informed consent form previously approved by the Ethics Committee School of Dental medicine University of Zagreb (13th September 2019. No: 05-PA-30-IX-9/2019).
3. Patients who do not take other local preparations for periodontitis treatment or toothpaste.
4. Patients who are willing to comply with the treatment and to follow instructions and be available for the duration of the study and are willing to come for follow up appointments according to study plan.

### 3.2. Exclusion criteria:

1. Patients who use other local oral therapy and those with complete dentures.
2. Non consented patients and those with the inability to participate due to any personal or objective reason mentioned.

### 3.3 Treatment Plan

### 1. at baseline:

- Medical and dental history
- Clinical assessment of periodontal pockets, plaques and orthopantomogram (a panoramic single image radiograph of the mandible, maxilla and teeth)
- Sampling: 0,5 ml unstimulated saliva collected by spitting method and paper point smears from the deepest pockets according to the orthopantomogram image
- Six-point probing to measure pocket depth (PD) and bleeding on probing (BoP)
- Instructions to patient how to brush teeth at home with Panaceo PMA-Zeolite toothpaste
- Plaque-free professional periodontal prophylaxis in the dental office
- Application of PMA-Zeolite demineralized water solution into deepest periodontal pockets

### 2. at the end of 10th week (end of PMA-Zeolite treatment)

- Sampling: 0,5 ml unstimulated saliva collected by spitting method and paper point smears from the same periodontal pockets as taken at baseline
- Six-point probing to measure PD and BoP
- All patients were given plaque-free professional prophylaxis as at baseline. Plaque status was again assessed.
- Application of PMA-Zeolite demineralized water solution into deepest periodontal pockets
- Assessment of the clinical status of the oral cavity

### 3.4. Follow-up after 12 week-period (2 weeks upon PMA-Zeolite treatment completion):

- Assessment of the clinical status of the oral cavity
- Assessment of subjective feeling of the patients (patients were required to respond according to their subjective feeling in the oral cavity: "I feel better, "I feel worse", "I feel the same")

### 3.5 Data Collection and Statistics

- Data from clinical measurements (PD, BoP, API) before and after each treatment; subjective patient state.
- Sample analysis: whole genome oral microbiome sequencing. All data were statistically analysed.
- Comparison of the clinical and oral microbiome status before and after PMA-Zeolite therapy expecting a change in clinical findings and bacterial diversity.
- Assessment of the subjective state of patients in the follow-up (12 weeks from baseline)

### 4. Materials and procedures

Small plastic sterile containers for saliva, paper points for periodontal smear samples, small container for liquid nitrogen, liquid nitrogen, sample transportation, masks, gloves were used for sample collection. Samples were stored in standard PBS solution (Phosphate buffered saline; sodium chloride, potassium chloride, potassium dihydrogen phosphate, and disodium hydrogen phosphate) at -80_{°}C until DNA extraction. For DNA extraction Chemagic 360 system was used at the Geneplanet (Ljubljana, Slovenia). For the oral microbiome sequencing at the Geneplanet, next-generation shotgun sequencing of the whole oral metagenome was performed by use of the platform: Novaseq 6000; Reads, Length: pair-end 150bp; Library preparation kit: NEB (New England Biolabs, UK) Next^{®} Ultra^{™} DNA Library Prep Kit; >2 Gb data/sample. The in-house and Kraken metagenomic sequence classification was used for identifications.

### 5. Statistical analysis

For assessment of clinical parameters in patients that completed the PMA-Zeolite therapy, the following tests were performed for BoP and PD: Fisher's Exact Test for Count Data, McNemar's Chi-squared test with continuity correction - McNemar's chi-squared tests.

Whole oral metagenome shotgun reads were mapped onto the reference human genome (hg37) using the bwa 0.7.17 aligner. Reads not mapped onto reference human genome were considered of microbial origin and were used to determine their taxonomy with the MetaPhlAn 3 program. As database of marker genes for taxonomic profiling mpa_v30_CHOCOPhlAn_201901 release was used. Unmapped reads were used as paired-end reads natively by MetaPhlAn (without joining them beforehand). MetaPhlAn taxonomic profiles of individual samples were merged using merge_metaphlan_tables.py script into a single taxonomic table. STAMP v 2.1.3. was used to determine statistical differences in obtained taxonomic profiles.

### 6. Results and discussion

### 6.1. Clinical status

The clinical study "The effect of PMA-Zeolite on the clinical and the microbiome state of periodontium in the treatment of periodontitis ", was performed at the School of Dental Medicine, University of Zagreb from 2.12.2019. until 7.7.2021. Out of 42 initially enrolled patients with *periodontitis chronica,* 36 subjects completed the clinical study successfully.

### Data on patients' drop-out

- 12 patients quitted the study in the very beginning during the recruiting, after the major earthquake in Zagreb in spring 2020 and did not come back for further examinations and follow-up even after direct telephone contacting.
- 13 patients did not comply with the treatment or quitted the treatment due to diagnosed Covid-19 disease or other declared 'private reasons' (only one patient specified the private reason as unexpected need for taking care of elderly family members).
- 1 patient did not provide clear data on treatment complying.
- 5 patients did not show up for control at the end of therapy (10th week).
- 6 control patients that received only standard periodontic prophylaxis, did not appear at the control examination after 10th week. Formally, these patients are noted as patients requiring further periodontic treatment. The usual drop-out of such patients that do not return to control appointment, occurs due to lack of disease improvement (lack of treatment therapy effect) that increases fear of therapy continuation.

### Patients' clinical condition before PMA-Zeolite therapy (baseline) was assessed as follows:

Inflammation, swelling, redness and bleeding of the gingiva was clearly visible. Deep periodontal pockets were visible as well and an unpleasant breath odor was present.

### Clinical description of patients' condition that completed the study after PMA-Zeolite therapy (10th week) was assessed by inspection of the oral cavity as follows:

- After the initial periodontal therapy and the therapy with PMA-Zeolite for 10 weeks, clinical improvement was observed in all patients, without exception, in the form of a reduction in the pocket depth (PD) on probing, an improvement in the condition of the gingiva where the appearance of the gingiva colour changed from red to pink (healthy state). A visible reduction in inflammation, swelling and sensitivity of the gingiva and surrounding mucosa was observed in all patients after the therapy. At the end of PMAZeolite treatment, patients were given instructions on further maintenance of oral hygiene of the oral cavity.

### Clinical condition of patients after the follow-up control period (12 weeks after baseline)

The clinical examination confirmed improvement of the periodontic state in the oral cavity, persistent reduced inflammation, reduction of bad breath and bleeding and the colour of the gingiva as pink (healthy).

A subjective assessment of the oral cavity condition was given in oral form from each patient. The question given to patients was: "How do you feel related to the condition in your oral cavity" and the answers given were:
- "I feel better" - 24 patients
- "I feel worse" - 7 patients
- "I feel the same" - 5 patients

### 6.2. Statistical analysis

### 6.2.1. Statistical analysis of clinical parameters for all patients that successfully completed the PMA-Zeolite-treatment cycle

Two parameters were analysed: (1) The **Bleeding on probing (BoP)** is a test/index that shows the degree of inflammation followed by bleeding, so it is also a motivational index because when the patient sees that the gingiva bleeds less after periodontal therapy, when brushing the teeth or spontaneously, a higher level of motivation to continue the therapy is achieved; (2) **pocket depth (PD)** parameter showing the depth from 3-6 mm (up to 3 mm normal/healthy pockets, 4 -5 mm shallow pockets and 6 mm deep pockets). The approximal plaque index (API) was not assessed as a clear indicator of PMA-Zeolite treatment as it correlates directly with the standard professional periodontal prophylaxis received at baseline of the study. The comparison by chi square test was done between the groups marked as: controls (K) vs. PMA-Zeolite-therapy (N) and PMA-Zeolitetherapy_start (N_S) vs. PMA-Zeolite-therapy_end (N_E), where N stays for PMA-Zeolitetherapy (Table 1). The results at the end of PMA-Zeolite-therapy showed a marked statistically significant difference towards improved values comparing baseline and the end of the treatment:
- BoP: Fisher's Exact Test for Count Data, p-value = 1.187e-09; McNemar's Chi-squared test with continuity correction - McNemar's chi-squared = 21.043, df = 1, p-value = 4.49e-06).
- PD: Fisher's Exact Test for Count Data, p-value = 4.684e-08; McNemar's Chi-squared test with continuity correction - McNemar's chi-squared = 14.062, df = 1, p-value = 0.0001768

**Table 1. Clinical parameters assessed in patients (N= 36) at the baseline and end of therapy. Control patients (N=6) that received only standard periodontic prophylaxis are presented at the end of the table separately. Categories for pocket depth (PD) statistical analysis were assessed as following: shallow+deep = DEEP; healthy+shallow=SHALLOW; healthy+shallow+deep=ALL. Patients were categorized in the following groups: N_S - start of the treatment with PMA-Zeolite; N_E - end of the treatment with PMA-Zeolite.**

| **Patient no.** | **BoP_N_S** | **BoP_N_E** | **API_N_S** | **API_N_E** | **PD_N_S** | **PD_N_E** |
|---|---|---|---|---|---|---|
| **PMA-ZEOLITE TREATMENT GROUP** | | | | | | |
| **1** | **3,4** | **2,3** | **+** | **+/-** | **>3** | **<3** |
| **2** | **3,4** | **3,4** | **+** | **-** | **>3** | **<3** |
| **3** | **3,4** | **2,3** | **+** | **-** | **>3** | **<3** |
| **4** | **3,4** | **2,3** | **+** | **-** | **>3** | **<3** |
| **5** | **2,3,4** | **3** | **+** | **+/-** | **>3** | **3** |
| **6** | **3,4** | **3** | **+** | **-** | **>3** | **<3** |
| **7** | **2,3,4** | **2,3** | **+** | **-** | **>3** | **2,3** |
| **8** | **3,4** | **2,3** | **+** | **-** | **>3** | **2,3** |
| **9** | **3,4** | **2,3** | **+** | **-** | **>3** | **<3** |
| **10** | **3,4** | **3** | **+** | **-** | **>3** | **3,4** |
| **11** | **3,4** | **3** | **+** | **-** | **>3** | **5,6** |
| **12** | **3,4** | **2,3** | **+** | **-** | **>3** | **3,4** |
| **13** | **3,4** | **2,3** | **+** | **+/-** | **>3** | **2,3** |
| **14** | **3,4** | **3** | **+** | **-** | **>3** | **<3** |
| **15** | **3,4** | **3** | **+** | **-** | **>3** | **<3** |
| **16** | **3,4** | **3** | **+** | **-** | **>3** | **<3** |
| **17** | **3,4** | **3,4** | **+** | **-** | **>3** | **<3** |
| **18** | **3,4** | **3** | **+** | **-** | **>3** | **<3** |
| **19** | **3,4** | **3** | **+** | **-** | **>3** | **<3** |
| **20** | **3,4** | **3** | **+** | **-** | **>3** | **3,4,5** |
| **21** | **3,4** | **2,3** | **+** | **-** | **>3** | **4,5** |
| **22** | **3,4** | **2,3** | **+** | **-** | **>3** | **3,4** |
| **23** | **3,4** | **2,3** | **+** | **-** | **>3** | **3,4** |
| **24** | **3,4** | **3,4** | **+** | **-** | **>3** | **<3** |
| **25** | **3,4** | **2** | **+** | **+/-** | **>3** | **<3** |
| **26** | **3,4** | **2** | **+** | **+/-** | **>3** | **<3** |
| **27** | **3,4** | **2,3** | **+** | **-** | **>3** | **<3** |
| **28** | **3,4** | **2,3** | **+** | **-** | **>3** | **2,3,4** |
| **29** | **3,4** | **2,3** | **+** | **-** | **>3** | **3,4** |
| **30** | **3,4** | **2,3** | **+** | **-** | **>3** | **2,3,4** |
| **31** | **3,4** | **2,3** | **+** | **-** | **>3** | **3** |
| **32** | **3,4** | **2,3** | **+** | **-** | **>3** | **3** |
| **33** | **3,4** | **2** | **+** | **-** | **>3** | **3,4** |
| **34** | **3,4** | **2,3** | **+** | **-** | **>3** | **2,3** |
| **35** | **3,4** | **2,3** | **+** | **-** | **>3** | **3,4** |
| **36** | **3,4** | **2,3** | **+** | **-** | **>3** | **3,4** |
| | | | | | | |

| **CONTROL GROUP** | | | | | | |
|---|---|---|---|---|---|---|
| 1 | 3,4 | * | + | * | >3 | * |
| 2 | 3,4 | * | + | * | >3 | * |
| 3 | 3,4 | * | + | * | >3 | * |
| 4 | 3,4 | * | + | * | >3 | * |
| 5 | 3,4 | * | + | * | >3 | * |
| 6 | 3,4 | * | + | * | >3 | * |

| | | | | | | |
|---|---|---|---|---|---|---|
| ** Control patients that received only standard periodontic prophylaxis did not come to control upon the 10-week period from the beginning of the study and the data is accordingly unavailable. The usual drop-out of such patients that do not return to control, occurs due to lack of disease improvement (lack of treatment therapy effect) that increases fear of therapy continuation.* | | | | | | |

### 6.2.2. Statistical analysis of clinical parameters for patients with sequencing results

Two parameters were analysed: (1) The **Bleeding on probing (BoP)** is a test/index that shows the degree of inflammation followed by bleeding, so it is also a motivational index because when the patient sees that the gingiva bleeds less after periodontal therapy, when brushing the teeth or spontaneously, a higher level of motivation to continue the therapy is achieved and (2) **pocket depth (PD)** parameter showing the depth from 3-6 mm (up to 3 mm normal/healthy pockets, 4 -5 mm shallow pockets and 6 mm deep pockets). The comparison by chi square test was done between the groups marked as: controls (K) vs. PMA therapy (N) and PMA-Zeolite-therapy_start (N_S) vs. PMA-Zeolite-therapy_end (N_E) (Table 2).

The groups were assessed on the base of the BoP (groups 1-4). None of the group comparisons in patients with sequencing data were statistically significant for the BoP parameter (p=0.65 for K vs N; p= 0.39 for N_S vs. N_E):

For the PD testing subjects were grouped into 3 groups based on the determined depth of the pockets - subjects with deep pockets, shallow pockets and subjects having both (ALL). Statistical testing for differences between therapy start (N_S) and therapy end (N_E) in patients with sequencing data did not show significant differences between the groups (p=0.14 for N_S vs. N_E).

**Table 2. Clinical parameters assessed in patients with sequencing data. Categories for pocket depth (PD) statistical analysis were assessed as following: shallow+deep = DEEP; healthy+shallow=SHALLOW; healthy+shallow+deep=ALL. Patients were categorized in the following groups: K - control; N - treatment with PMA-Zeolite; N_S - start of the treatment with PMA-Zeolite; N_E - end of the treatment with PMA-Zeolite.**

| **No.** | **ID** | **BoP** | **Bleeding** | **Group** | **Timepoint** | **Deepness of the pocket** |
|---|---|---|---|---|---|---|
| 1 | N_48_E_X | 3,4 | high | N | N_E | DEEP |
| 2 | N_40_S_X | 3,4 | high | N | N_S | DEEP |
| 3 | N_59_E_X | 3,4 | high | N | N_E | DEEP |
| 4 | N_41_S_X | 3,4 | high | N | N_S | SHALLOW |
| 5 | N_47_E_X | 3,4 | high | N | N_E | DEEP |
| 6 | N_59_S_X | 3,4 | high | N | N_S | DEEP |
| 7 | N_39_S_X | 3,4 | high | N | N_S | DEEP |
| 8 | N_56_E_X | 3,4 | high | N | N_E | DEEP |
| 9 | N_23_S_X | 1,2,3,4 | full | N | N_S | DEEP |
| 10 | N_25_S_X | 3,4 | high | N | N_S | DEEP |
| 11 | N_4_S_X | 2 | low | N | N_S | ALL |
| 12 | N_19_S_X | 1,2,3,4 | full | N | N_S | ALL |
| 13 | N_26_S_X | 2,3,4 | high | N | N_S | ALL |
| 14 | N_41_E_X | 3,4 | high | N | N_E | SHALLOW |
| 15 | N_47_S_X | 3,4 | high | N | N_S | DEEP |
| 16 | N_39_E_X | 3,4 | high | N | N_E | DEEP |
| 17 | N_54_E_X | 3,4 | high | N | N_E | SHALLOW |
| 18 | N_23_E_X | 1,2,3,4 | full | N | N_E | DEEP |
| 19 | N_67_E_B | 3,4 | high | N | N_E | ALL |
| 20 | N_66_E_PP | 3,4 | high | N | N_E | ALL |
| 21 | N_46_E_B | 2,3 | avg | N | N_E | DEEP |
| 22 | N_66_S_PP | 3,4 | high | N | N_S | ALL |
| 23 | N_67_S_PP | 3,4 | high | N | N_S | ALL |
| 24 | N_65_E_PP | 3,4 | high | N | N_E | SHALLOW |
| 25 | N_66_S_B | 3,4 | high | N | N_S | ALL |
| 26 | N_61_E_PP | 2,3 | avg | N | N_E | SHALLOW |
| 27 | N_67_S_B | 3,4 | high | N | N_S | ALL |
| 28 | N_67_E_PP | 3,4 | high | N | N_E | ALL |
| 29 | N_63_E_B | 3,4 | high | N | N_E | ALL |
| 30 | N_66_E_B | 3,4 | high | N | N_E | ALL |
| 31 | N_65_E_B | 3,4 | high | N | N_E | SHALLOW |
| 32 | N_64_E_B | 3,4 | high | N | N_E | SHALLOW |
| 33 | N_63_E_PP | 3,4 | high | N | N_E | ALL |
| 34 | N_64_E_PP | 3,4 | high | N | N_E | SHALLOW |
| 35 | N_62_E_B | 3,4 | high | N | N_E | ALL |
| 36 | N_61_E_B | 2,3 | avg | N | N_E | SHALLOW |
| 37 | K_11_S_X | 3,4 | high | K | K | |
| 38 | K_10_S_X | 3,4 | high | K | K | |
| 39 | K_5_S_X | 3,4 | high | K | K | |
| 40 | K_7_S_X | 3,4 | high | K | K | |
| 41 | K_21_S_X | 3,4 | high | K | K | |
| 42 | K_16_S_X | 3,4 | high | K | K | |

Although there was no statistically significant difference in clinical indices (BoP and PD) before and after 10 weeks of PMA-Zeolite therapy, clinical improvements after specialist visual exam were observed and documented officially by clinicians and confirmed by subjective impression of patients at the follow-up.

### 6.3. Microbiome analysis

In all patients with diagnosed *periodontitis chronica,* specifically enriched phyla were *Proteobacteria, Firmicutes, Bacterioidetes, Actinobacteria, Synergistetes, Spirochaetes, Fusobacteria and Chlorofelxi* (Welch's t-test, two-sided, p<0.05) (Figure 1). This is in line with previously established content of the human oral microbiome and literature data on periodontitis patients https://www.ncbi.nlm.nih.gov/pmc/articles/PMC2944498/pdf/0542-10.pdf, https://www.frontiersin.org/articles/10.3389/fcimb.2020.604596/full https://www.ncbi.nlm.nih.gov/pmc/articles/PMC9103139/ https://saludpublica.uchile.cl/documentos/ir-a-microbial-signatures-of-health-gingivitis-andperiodontitis_174395_0_5836.pdf

The Human Microbiome Project found that the main genus among intraoral sites, at greater than 10% abundance and present in more than 75% of samples, was *Streptococcus.* Other core members with greater than 1% abundance across at least 80% of samples from 1 or more sites, included those bacteria from the family *Pasteurellaceae,* those from the genera *Gemello, Veillonello, Prevotella, Fusobacterium, Porphyromonas, Neisseria, Copnocytophogo, Corynebacterium, and Actinomyces,* and those from the orders *Lactobacillales and Lachnospiraceae.* Still, the exact general composition of the oral microbiota in individuals is not subject to standardization and is inherently difficult due to various factors affecting the oral microbiome content all the time (i.e. food intake, oral hygiene, daily fluctuations, individual differences etc.). (see Lea Sedghi, Vincent DiMassa, Anthony Harrington, Susan V. Lynch, Yvonne L. Kapila (2021) The oral microbiome: Role of key organisms and complex networks in oral health and disease, Periodontology 2000 87(1): 107-131; Sharma N, Bhatia S, Sodhi AS, Batra N. Oral microbiome and health. AIMS Microbiol. 2018 Jan 12;4(1):42- 66. doi: 10.3934/microbiol.2018.1.42).

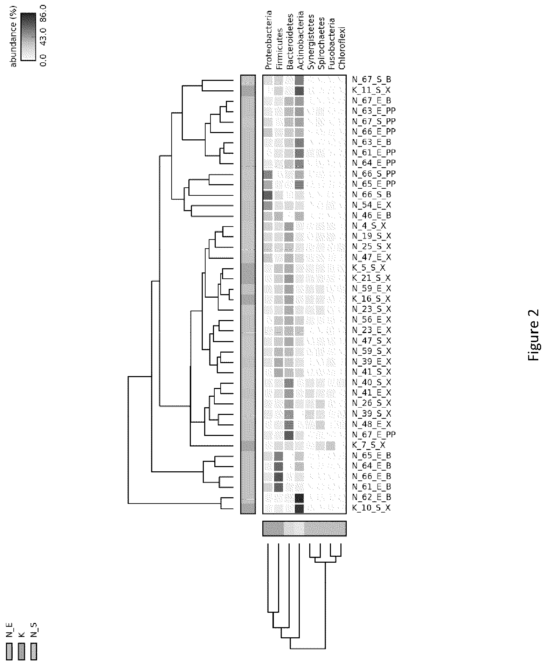

The results are shown in Figure 2.

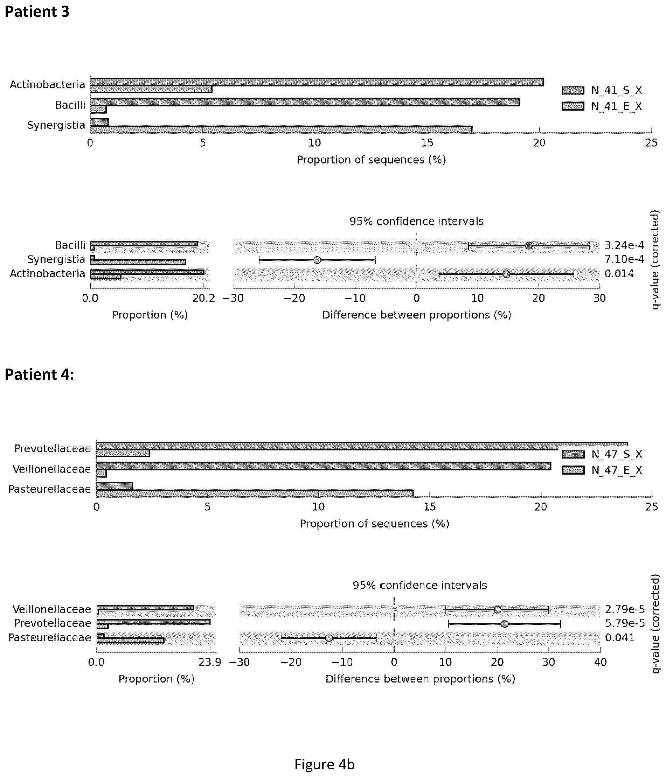

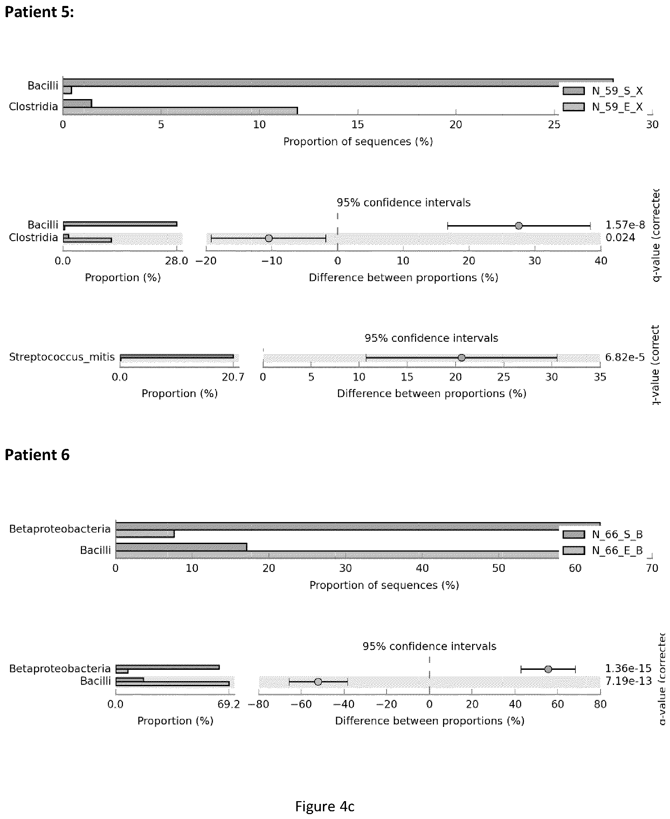

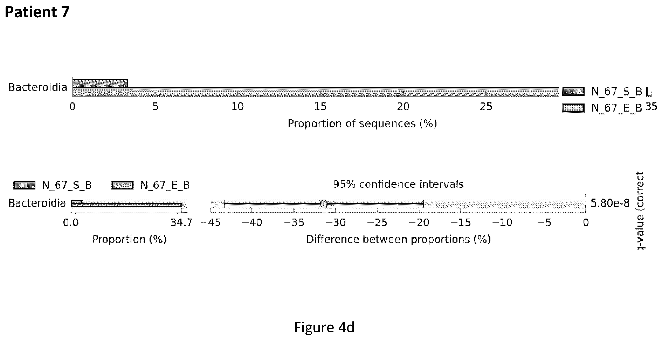

Individual statistically relevant class differences were obtained for 7 patients (two-sided Fisher's exact test with Benjamini-Hochberg FDR correction was used, p<0.05) showed a highly individualized response in class comparison due to large oral microbiome variations already documented in the literature. These individual data is presented for each patient in Figure 4.

In a general comparison of the microbiome status in **ALL tested patients' samples** before and after therapy, significant results (Welch's test, two-sided, p<0.05 - no correction, orange before therapy, blue -after therapy) as shown in Figure 3.

In summary, the observed improvements in the clinical observations of patients diagnosed with *Periodontitis chronica* treated with PMA-Zeolite for 10 weeks, clearly point to positive effects of PMA-Zeolite treatment on the inflammatory status of the oral cavity, whereby the treatment decreased the inflammation symptoms (redness of the mucosa and mucosa degradation manifested through decreased bleeding on probing (BoP) and pocket depth (PD) status parameters). PMA-Zeolite proved effective in decreasing the abundance of some established oral markers of dysbiosis and/or inflammation.

### 7. Conclusions

The results of PMA-Zeolite treatment have shown clear improvement in clinical periodontal indices after local application of PMA-Zeolite toothpaste and rinsing of pathological periodontal pockets with PMA-Zeolite demineralized water solution in all patients after 10 weeks of PMA-Zeolite treatment. The clinical results at the end of PMA-Zeolite therapy showed a marked statistical difference towards improved values as follows:

For the BoP: Fisher's Exact Test for Count Data, p-value = 1.187e-09; McNemar's Chi-squared test with continuity correction - McNemar's chi-squared = 21.043, df = 1, p-value = 4.49e-06).

For the PD: Fisher's Exact Test for Count Data, p-value = 4.684e-08; McNemar's Chi-squared test with continuity correction - McNemar's chi-squared = 14.062, df = 1, p-value = 0.0001768

The improved clinical state and subjective feeling were confirmed at the follow-up as well. The identified microbiome changes also point to the effectiveness of PMA-Zeolite-based preparations on the entire periodontal microbiome status in the treatment of periodontal disease.

The identified enriched phylum in patients with diagnosed *periodontitis chronica* were *Proteobacteria, Firmicutes, Bacterioidetes, Actinobacteria, Synergistetes, Spirochaetes, Fusobacteria and Chlorofelxi* (ANOVA, Tukey-Kramer, eta squared, p<0.05). This is in line with previously established content of the human oral microbiome and literature data on parodontids patients. Indeed, the Human Oral Microbiome Database (HOMD, www.homd.org ) includes 619 taxa in 13 phyla, as follows: *Actinobacteria, Bacteroidetes, Chlamydiae, Chloroflexi, Euryarchaeota, Firmicutes, Fusobacteria, Proteobacteria, Spirochaetes, SR1, Synergistetes, Tenericutes, and TM7.* Moreover, in periodontitis the following phyla are correlated with the symptoms: *Spirochaetes, Synergistetes and Bacteroidetes.*

The statistically relevant microbiome analysis comparison of ALL samples before and after PMA-Zeolite treatment confirmed specifically that (Welch's test, two-sided, p<0.05 - no correction):
- PMA-Zeolite treatment decreased the abundance of the class Deltaproteobacteria that are found to be increased in disease and have been identified by some authors as a possible marker of microbiota instability, thus, predisposing to disease onset;
- PMA-Zeolite treatment decreased the abundance of the family Desulfobulbaceae that is found to play a role in inflammation within the oral cavity of patients with parodontosis;
- PMA-Zeolite treatment increased the abundance of the family Lachnospiraceae, that has dual roles in different pathogeneses but in connection with PMA-Zeolite treatment it may be evaluated as a potential signature for reduced histamine content in the oral cavity;
- PMA-Zeolite treatment decreased the abundance of the genus Desulfobulbus that is found to play a role in inflammation within the oral cavity of patients with parodontosis;
- PMA-Zeolite treatment increased the abundance of the genus Oribacterium;
- PMA-Zeolite treatment decreased the abundance of the species Desulfobulbus oralis an established oral pathobiont that can trigger a proinflammatory response in oral epithelial cells, suggesting its direct role in the development of periodontal disease.
- PMA-Zeolite treatment decreased the abundance of the species Gemella bergeri that can become pathogenic and cause infections in patients with poor oral hygiene
- PMA-Zeolite treatment increased the abundance of the species Oribacterium sp. (not associated so far with periodontitis, mainly studied in other oral diseases)

**Table 3. Summarized significantly relevant results (p<0.05) of the oral microbiota changes in periodontic patients upon 10-week treatment with PMA-Zeolite**

| **Taxonomy level** | **Oral microbiome representative** | **Abundance change upon PMA-zeolite treatment** |
|---|---|---|
| **Class** | **Deltaproteobacteria*** | **↓** |
| **Family** | **Desulfobulbaceae *** | **↓** |
| **Family** | **Lachnospiraceae*** | **↑** |
| **Genus** | **Desulfobulbus *** | **↓** |
| **Genus** | **Oribacterium** | **↑** |
| **Species** | **Desulfobulbus oralis *** | **↓** |
| **Species** | **Gemella bergeri *** | **↓** |
| **Species** | **Oribacterium sp.** | **↑** |

| | | |
|---|---|---|
| ** established indicators of inflammation and oral dysbiosis in oral disease* | | |

In summary, the observed improvements in the clinical observations of patients diagnosed with *Periodontitis chronica* treated with PMA-Zeolite for 10 weeks, clearly point to positive effects of PMA-Zeolite treatment on the inflammatory status of the oral cavity, whereby the treatment decreased the inflammation symptoms (redness of the mucosa and mucosa degradation manifested through decreased bleeding on probing (BoP) and pocket depth (PD) status parameters). While the standard periodontic prophylaxis along with oral hygiene instructions are successful in prevention of periodontal diseases, treatment of inflammation, especially chronic inflammation in the established periodontal disease, requires additional procedures that, beside cleaning of the plaques, includes systemic or local antibiotic treatment as well30. Considering the results of the microbiome analysis in this study, it may be concluded that a microbiome dysbiosis (particularly changes in established bacteria taxa) occurs in *periodontic chronica* patients, which corroborates the treatment efficacy of agents directed to management of the oral dysbiosis status related to inflammation. PMA-Zeolite proved effective in this particular application as it decreased the abundance of some established oral markers of dysbiosis and/or inflammation and may thus be regarded as an alternative and safe therapeutic approach alone or in combination with antibiotic treatment for periodontic patients.

The above results convincingly show the effect of a micronisation activated clinoptilotite in the treatment of chronic periodontitis. It is evident that also other inflammatory conditions of the oral cavity can successfully be treated by the application of micronisation activated clinoptilotite. Inflammation, as the response of the body to injury, may be acute or chronic. If the inflammation is not cured it leads to diverse pathologies. In particular, chronic inflammatory illnesses such as diabetes, arthritis, and heart disease may have an impact on the periodontium. Periodontal inflammation as the most common inflammation of the oral cavity is associated with the accumulation of bacteria at the tooth surface leading to a host response generating inflammatory cell infiltration. Regulation of immune-inflammatory mechanisms in oral disease is particularly coordinated by macrophages that are crucial for resolving the inflammation (Hasturk H, Kantarci A, Van Dyke TE. Oral inflammatory diseases and systemic inflammation: role of the macrophage. Front Immunol. 2012 May 16;3:118. doi: 10.3389/fimmu.2012.00118. PMID: 22623923; PMCID: PMC3353263.). Accordingly, PMA-zeolite may be used to treat generally the oral cavity inflammations as it has been previously assesed that clinoptololite acts as nonspecific immunostimulant similar to superantigens (Kraljevi 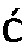 Paveli 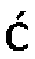 S, Simovi 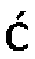 Medica J, Gumbarevi 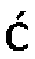 D, Filo 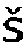 evi 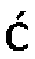 A, Pr 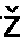 ulj N, Paveli 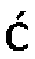 K. Critical Review on Zeolite Clinoptilolite Safety and Medical Applications in vivo. Front Pharmacol. 2018;9:1350.). In addition, phagocytic cells, mainly macrophages, are activated by zeolites enhancing phagocytosis and reactive oxygen species production (Gr 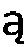 dzki Z, Jarosz 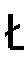 , St 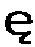 pie 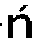 -Py 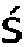 niak D, Marek A.

### Example 2: Exemplary formulation

In the following, an exemplary formulation of a zeolite toothpaste is shown.

| **INCI** | **Quantitative** |
|---|---|
| Glycerin | B |
| Aqua | B |
| Hydrated Silica | C |
| Zeolite | D |
| Aloe Barbadensis Leaf Juice* | E |
| Mentha Piperita Water* | E |
| Sodium Coco-Sulfate | E |
| Xanthan Gum | E |
| Zinc Oxide | E |
| Mentha Spicata Herb Oil | F |
| Mentha Arvensis Leaf Oil* | F |
| Chondrus Crispus Powder | F |
| Stevia Rebaudiana Leaf/Stern Extract | G |
| Citric Acid | H |
| Potassium Sorbate | H |
| Sodium Benzoate | H |

| **Allergene** | **Quantitative** |
|---|---|
| Limonene** | G |
| Linalool** | H |

| | |
|---|---|
| * certified organic ** natural fragrance *** made using organic ingredients | |

Ranges of concentration:

| | | | | | | | |
|---|---|---|---|---|---|---|---|
| A1 | 75-100% | A2 | 50-75% | B | 25-50% | C | 10-25% |
| D | 5-10% | E | 1-5% | F | 0.1-1% | G | < 0.1% |
| H | Traces of Substances | | | | | | |

### INFO for packaging design:

The order of all INCI which contain more than 1% of the total amount is fixed, as in the example below. For INCI with content below 1% (incl. range "F"), the order list is optional (see list above).

### Declaration of the ingredients on the toothpaste:

Glycerin, Aqua, Hydrated Silica, Zeolite, Aloe Barbadensis Leaf Juice*, Mentha Piperita Water*, Sodium Coco-Sulfate, Xanthan Gum, Zinc Oxide, Mentha Spicata Herb Oil, Mentha Arvensis Leaf Oil*, Chondrus Crispus Powder, Stevia Rebaudiana Leaf/Stem Extract, Citric Acid, Potassium Sorbate, Sodium Benzoate, Limonene**, Linalool**.
* certified organic
** natural fragrance
*** made using organic ingredients

The toothpaste has to be stored at room temperature (15°C to 25°C).

## Claims

1. An oral care composition comprising a micronization-activated clinoptilotite and one or more pharmaceutically acceptable excipients for use in the treatment and/or prevention of an oral inflammatory condition and/or an oral infection.

2. The oral care composition for the use of claim 1, wherein the micronization-activated clinoptilotite is a zeolite particle composition obtained by a process according to EP 3 329 926.

3. The oral care composition for the use of any of claims 1 or 2, wherein the oral inflammatory condition is an acute or chronic periodontal disease, a peri-implant disease, an oral wound and/or an aphthous lesion.

4. The oral care composition for the use of claim 3, wherein the periodontal disease is gingivitis, stomatitis, oral mucositis and/or periodontitis.

5. The oral care composition for the use of any of claims 1 to 4, wherein said oral infection is a bacterial, viral or fungal infection.

6. The oral care composition for the use of any of claims 1 to 5, wherein said composition is selected from a mouthwash, a toothpaste, a powder, a dental gel, a chewing gum, a dissolvable, partially dissolvable or non-dissolvable film or strip or a dental floss.

7. The oral care composition for the use of any of claims 1 to 6, wherein the composition further comprises one or more of a fluoride ion source, breath freshening agents, antiplaque agents, analgesics, nutrients, abrasives, surfactants, thickening agents, buffers, preservatives and antibiotic and anti-caries agents.

8. The oral care composition for the use of any of claims 1 to 7, wherein the composition is a toothpaste having a content of micronization-activated clinoptilotite of 2% wt/wt to 10% wt/wt.

9. The oral care composition for the use of any of claims 1 to 8, wherein the composition is a package containing powdered micronization-activated clinoptilotite for the preparation of a suspension for oral cavity rinsing including periodontal pockets.

10. The oral care composition for the use of any of claims 1 to 9, wherein the composition is administered in a dosage from 0.01 to 1000 mg/kg of body weight, preferably from 0.1 to 500 mg/kg of body weight, more preferably from 10 to 100 mg/kg of body weight.
